# EUROPEAN PATENT APPLICATION

(11) **EP 1 479 408 A1**
(43) Date of publication of application: **24.11.2004**
(21) Application number: 04011858.0
(22) Date of filing: 19.05.2004
(51) Int. Cl.: A61M 25/06

(54) **Needle shield with wings**

(30) Priority: 23.05.2003 US 444537
(71) Applicant: Becton Dickinson and Company, Franklin Lakes, New Jersey 07417 (US)
(72) Inventor: Swenson, Kirk D., North Caldwell, New Jersey 07006 (US); Wilkinson, Bradley, North Haledon, New Jersey 07508 (US); Crawford, Jamieson, New York, New York 10025 (US); Niermann, Volker, Bound Brock, New Jersey 08805 (US)
(74) Representative: von Kreisler Selting Werner

(57) **Abstract**

A shielding medical device includes a needle cannula (18), a hub (22), a release element (60), a needle shield (40), and a biasing member (80). The hub has a proximal end, a distal end, and a passageway in fluid communication with the needle cannula. The distal end of the hub supports the needle cannula. The release element is fixed to the hub. The needle shield is hollow and has a proximal end, a distal end, and a pair of wings extending laterally from opposing sides. The proximal end of the needle shield is removably secured to the release element. The needle shield is axially movable with respect to the needle cannula from a first position in which the needle cannula is exposed to a second position in which the needle cannula is shielded within the needle shield. The biasing member is used to move the needle shield from the first to second positions.

## Description

### BACKGROUND OF THE INVENTION

### 1. Field of the Invention

The present invention relates to a medical device for safe and convenient handling of used needle cannulas. More particularly, the present invention relates to a medical needle device, preferably winged needle device, that is adapted to automatically shield a needle cannula after it is used in a medical procedure.

### 2. Description of Related Art

Blood collection sets or intravenous (IV) infusion sets typically include a needle cannula having a proximal end, a distal end with a puncture tip, and a lumen extending therebetween. The proximal end of the needle cannula is mounted to a plastic hub having a central passage that communicates with the lumen in the needle cannula. A thin flexible thermoplastic tube is connected to the hub and communicates with the lumen in the needle cannula. The end of the plastic tube remote from the needle cannula may include a fixture for connecting the needle cannula to a separate medical device, such as a holder, a blood collection tube, and the like.

In order to reduce the risk of incurring an accidental needle-stick wound, protection of used needle cannulas becomes important. With concern about infection and transmission of diseases, methods and devices to cover used needle cannulas have become important and in great demand. For example, some needle assemblies commonly employ a safety shield that may be moved into shielding engagement with a used needle cannula without risking an accidental needle stick.

U.S. Patent Nos. 5,085,639; 5,088,982; and 5,154,699 to Ryan disclose safety winged needle devices for use with blood collection sets or infusion sets. The safety needle devices of these patents include an inner tube and an outer tube having cooperating surfaces in contact with each other. The cooperating surfaces have mating grooves and ramps. By applying a force in opposing directions on the inner and outer tubes, the outer tube is moved forcibly along the ramps and into engagement with the grooves of the inner tube, thereby moving the outer tube, and therefore the outer shield, into a shielding position in place about the needle tip. However, the cooperating surfaces of such outer and inner tubes provide a frictional engagement that requires much force to overcome. Moreover, maintaining an appropriate grip on the safety needle device to forcibly move the outer and inner tubes apart to the fully shielded position can be difficult due to the profile of the safety needle device and the length of required travel to the shielded position.

U.S. Patent No. 5,505,711 discloses a winged injector needle assembly including a needle cannula attached to a hub and a holder forming a shield portion. The hub is slidable within the shield portion. The needle assembly also includes a latching mechanism between the hub and the shield portion to maintain the hub in a forward position with the needle cannula extending from the shield portion. Upon grasping of and release of the latching mechanism, the shield portion may be moved forward to shield the needle cannula. U.S. Patent No. 5,928,199 discloses a similar forward shielding needle assembly, which further includes a lock for locking the shield portion in the forward position shielding the needle cannula. Release of the latching mechanism is awkward due to the relation between the latching mechanism extending between the shield portion and the hub. Further, this device requires the operator to exert a substantial force to shield the needle cannula, and is prone to rotation of the needle cannula within the shield portion during the shielding operation.

U.S. Patent Nos. 5,779,679 and 6,210,371 to Shaw disclose winged IV sets with a retracting needle assembly adapted to automatically retract a needle cannula within a safety shield upon release of a latch. The winged IV sets include a pair of latch wings associated with an outer shield that hold the needle assembly in an unretracted position, and which may be released to cause the needle assembly to be retracted within the outer shield. A spring drives the needle assembly rearward to the retracted position within the safety shield. However, the spring extends entirely around the needle assembly during and after retraction, which may cause complications during retraction. Also, since retraction of such a device is achieved by holding the safety shield, the user does not hold the needle cannula during retraction and therefore does not maintain control over the needle cannula. Thus, if the latch is released while the needle cannula is inserted in a patient, the spring force may cause an undesirable partial or full retraction of the needle cannula, instead of merely passively activating the device to allow for complete retraction upon removal from the patient.

In view of the foregoing, there is a continuing need for a shielding medical needle device adapted to shield a used needle cannula once a medical procedure is completed.

### SUMMARY OF THE INVENTION

The present invention relates generally to a shielding medical needle device for shielding used needle cannulas. The shielding medical needle device generally includes a needle cannula, a hub, a release element, a needle shield, and, preferably, a biasing member. The hub includes a proximal end, a distal end and a passageway extending therethrough in fluid communication with the needle cannula. The distal end of the hub supports the needle cannula. The release element is fixed to the hub. The needle shield is generally hollow and includes a proximal end, a distal end and a pair of wings extending laterally from opposing sides thereof. The proximal end of the needle shield is removably secured to the release element with at least a grippable or graspable portion of the hub extending out from the proximal end of the needle shield. The needle shield is axially movable with respect to the needle cannula from a first position in which the needle cannula is exposed to a second position in which the needle cannula is shielded within the needle shield. The biasing member extends between the distal end of the hub and the proximal end of the needle shield. In operation, activation of the release element releases the proximal end of the needle shield from securement, thereby causing the biasing element to axially urge the needle shield from the first position toward the second position.

The biasing member may be a spring. The outer surface of the hub and the inner surface of the needle shield may interact such that axial rotation of the hub with respect to the needle shield is inhibited during movement of the needle shield from the first position toward the second position. For example, the hub and the needle shield may have non-circular cross-sectional shapes, thereby inhibiting axial rotation of the hub with respect to the needle shield during movement of the needle shield from the first position toward the second position. The inner surface of the needle shield may also include splines for inhibiting the axial rotation of the hub with respect to the needle shield during movement of the needle shield from the first position toward the second position.

The medical device may include a flexible tube extending from the proximal end of the hub. The medical device may further include a locking element for securing the needle shield in the second position. The locking element may include a pair of flexible locking arms at the distal end of the hub. Each pair of locking arms may include a hook for releasable engagement with a corresponding detent in the proximal end of the needle shield.

The release element may include at least one flexible locking arm extending distally from the hub and include a hook for releasable engagement with a corresponding detent in the proximal end of the needle shield. The release element may further include a pair of flexible locking arms extending distally from the hub. Each pair of locking arms may include a hook for releasable engagement with a corresponding detent in the proximal end of the needle shield. The at least one flexible locking arm may extend distally from the hub to form a pocket between the flexible locking arm and an outer surface of the hub adjacent the distal end of the hub. The pocket may accommodate the biasing member. In such an embodiment, the medical device may further include a locking element including at least one flexible locking arm at the distal end of the hub having a hook for releasable engagement with the detent in the proximal end of the needle shield when the needle shield is in the second position.

The release element may alternatively comprise a rotational locking engagement between the hub and the proximal end of the needle shield. A rotatable collar may extend from the hub. The rotatable collar may be independently rotatable with respect to the hub. The rotatable collar may be in releasable engagement with the proximal end of the needle shield when the needle shield is in the first position. The rotational locking engagement may be a threaded engagement.

The release element may also comprise at least one pivot arm extending laterally along the hub. The pivot arm may be in releasable engagement with the proximal end of the needle shield. The release element may further include a pair of pivot arms extending laterally along opposing outer sides of the hub. The pair of pivot arms may be in releasable engagement with corresponding surfaces on the proximal end of the needle shield.

In yet another embodiment, the medical device of the present invention includes a needle cannula, a hub, a needle shield, and a biasing member. The hub includes a proximal end, a distal end and a passageway extending therethrough in fluid communication with the needle cannula. The distal end of the hub supports the needle cannula. The needle shield is generally hollow and includes a proximal end, a distal end and a pair of wings extending laterally from opposing sides thereof. The needle shield is adapted to encompass the hub with at least a portion of the hub extending from the proximal end of the needle shield. The needle shield is axially movable with respect to the needle cannula from a first position in which the needle cannula is exposed to a second position in which the needle cannula is shielded within the needle shield. The biasing member extends between the hub and the needle shield and exerts a biasing force to urge the needle shield distally toward the second position. In operation, when the needle shield is in the first position the shield and the hub exhibit a mutual friction greater than the biasing force exerted by the biasing member when the needle shield is in the first position.

The mutual friction between the shield and the hub may be in the range of about 120% to about 1000% of the biasing force exerted by the biasing element. Desirably, the mutual friction is provided through surface texture on at least one of an outside surface of the hub or an inside surface of the needle shield. The portion of the hub that extends from the proximal end of the needle shield may include a surface grip and the needle shield may include a surface grip, such that opposing forces applied against the surface grip of the hub and the surface grip of the needle shield releases the mutual friction between the hub and the shield and the biasing element causes axial movement of the needle shield toward the second position.

The medical device may further include a locking element for securing the needle shield in the second position. An inner surface of the needle shield may include a first surface feature at the proximal end thereof and a second surface feature at the distal end thereof. The external surface of the hub may include a surface feature for interference engagement with the first surface feature of the needle shield when the needle shield is in the first position for retaining the needle shield in the first position, and for interference engagement with the second surface feature of the needle shield when the needle shield is the second position for securing the needle shield in the second position. The first and second surface features of the needle shield may comprise first and second detents, respectively, and the surface feature of the hub may comprise a bump for respective interference engagement with the first and second detents of the needle shield.

Further details and advantages of the present invention will become apparent from the following detailed description when read in conjunction with the drawings, wherein like parts are designated with like reference numerals and lower case letters are included where necessary to identify specific embodiments of the invention.

### DESCRIPTION OF THE DRAWINGS

FIG. 1 is a longitudinal cross sectional view of a medical device in accordance with the present invention showing a needle shield of the medical device locked in a first, retracted position proximate to a needle hub of the medical device;

FIG. 2 is a longitudinal cross sectional view of the medical device of FIG. 1 showing the needle shield in a second, extended position;

FIG. 3A is a transverse cross sectional view of the medical device of FIG. 1 showing the interaction between the needle hub and needle shield according to one embodiment of the present invention;

FIG. 3B is a transverse cross sectional view of the medical device of FIG. 1 showing the interaction between the needle hub and needle shield according to another embodiment of the present invention;

FIG. 4 is a longitudinal cross sectional view of another embodiment of the medical device of the present invention including a rotatable collar for locking the needle shield in the first position;

FIG. 5 is a longitudinal cross sectional view of the medical device of FIG. 4 showing the needle shield in the second, extended position;

FIG. 6 is a longitudinal cross sectional view of yet another embodiment of the medical device of the present invention including pivot arms on the needle hub for locking the needle shield in the first position;

FIG. 7 is a longitudinal cross sectional view of the medical device of FIG. 6 showing the needle shield in the second, extended position;

FIG. 8 is a longitudinal cross sectional view of the medical device according to a still further embodiment of the present invention, wherein the needle shield is locked in the first position by frictional interference between the needle hub and the needle shield;

FIG. 9 is a longitudinal cross sectional view of the medical device of FIG. 8 showing the needle shield locked in the second position by frictional interference between the needle hub and needle shield; and

FIG. 10 is a perspective view of the medical device of the present invention incorporated as part of a blood collection set.

### DETAILED DESCRIPTION

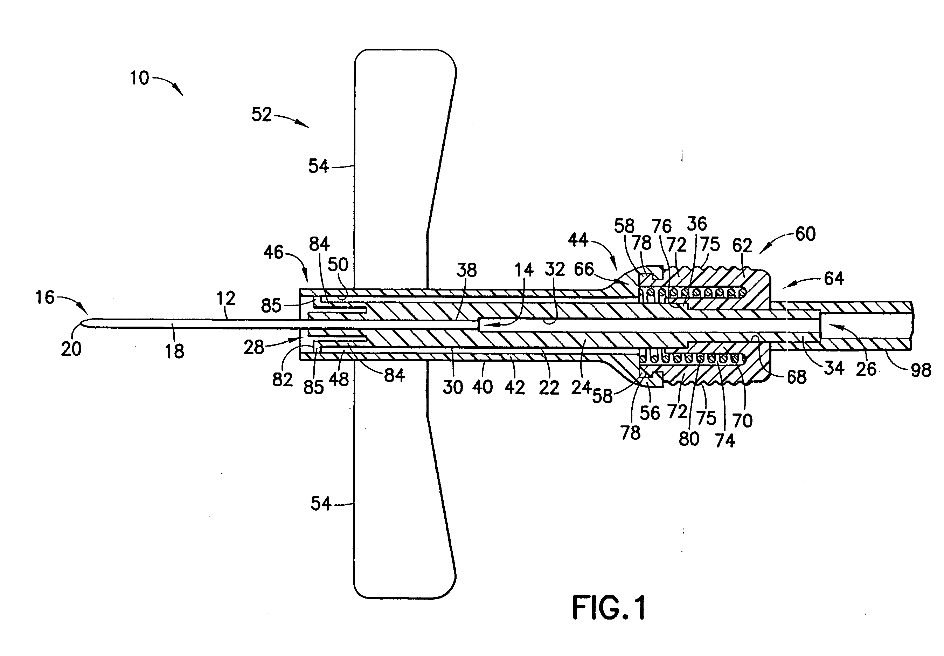

FIGS. 1-10 generally illustrate several embodiments of a medical device **10** according to the present invention. The several embodiments of the medical device **10** may be used as part of a fluid collection set used to collect blood or other fluids from the body of a human or animal. The medical device **10** of the present invention is generally a shieldable device adapted to enclose or surround a used needle cannula at the end of a fluid collection procedure. Preferably, the medical device **10** includes a portion that is automatically biased to a safety, needle-enclosing position upon activation by a user of the medical device **10**, as discussed in detail hereinafter.

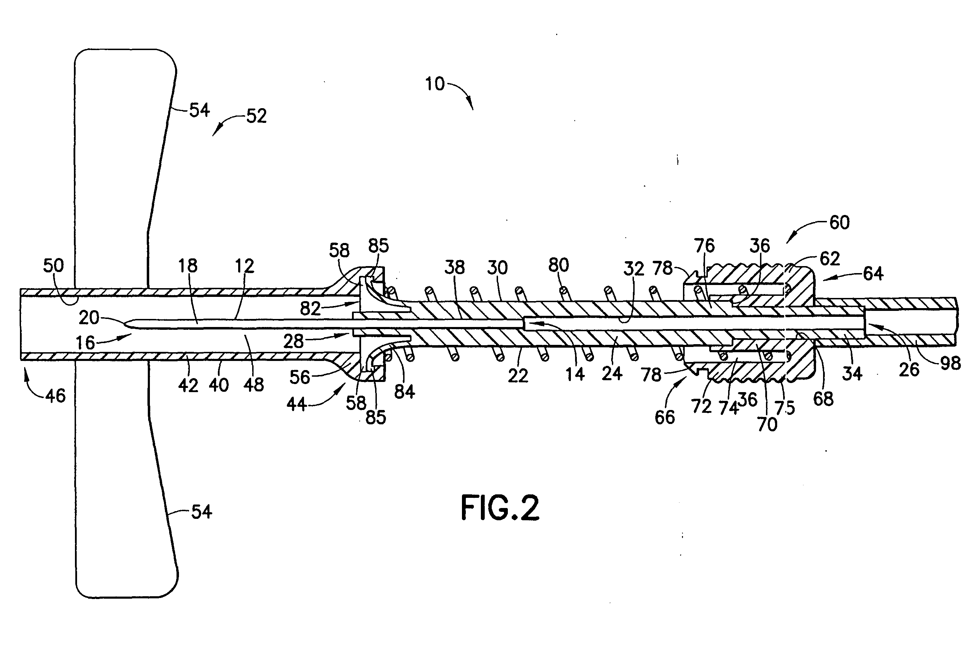

Referring to FIGS. 1 and 2, a first embodiment of the medical device **10** is shown. The medical device **10** generally includes a needle cannula **12**, a hub **22**, a needle shield **40**, a release element **60** releasably connecting the hub **22** and needle shield **40** and a biasing member **80**, for actuating (i.e., moving) the needle shield **40** to the safety, needle-enclosing position or configuration. The hub **22** is adapted for connection to a receptacle (not shown) of, for example, a blood collection set by way of a flexible tube **98** by means and procedures known in the art.

The needle cannula **12** includes a proximal end **14** and distal end **16** and defines a lumen **18** extending from the proximal end **14** to the distal end **16.** The distal end **16** of the needle cannula **12** is beveled to define a sharp puncture tip **20** suitable for venipuncture into the blood vessel of a human or animal. The puncture tip **20** is preferably designed to provide ease of insertion and minimal discomfort during venipuncture. A removable protective cover (not shown) may be positioned over the distal end **16** of the needle cannula **12** to prevent accidental needle-stick wounds prior to using the medical device **10** in a medical procedure and to protect the puncture tip **20** prior to use.

The hub **22** is preferably a unitary structure, which is desirably molded from a thermoplastic material. The hub **22** has a generally tubular-shaped body **24** with a proximal end **26** and a distal end **28**. The body **24** has an outer surface **30** and defines an internal passageway or lumen **32** extending from proximal end **26** to distal end **28**. The passageway **32** communicates with the lumen **18** defined in the needle cannula **12** to enable fluid, such as blood, to pass through the medical device **10** and to the tube **98** connecting the medical device **10** to the blood collection receptacle.

The proximal end **14** of needle cannula **12** is received within and supported by the distal end **28** of the body **24**. Particularly, the proximal end **14** of the needle cannula **12** is disposed in the passageway **32**, with the distal end **16** of needle cannula **12** projecting outward from the distal end **28** of the body **24**. The needle cannula **12** is preferably secured to the hub **22** through the use of an appropriate medical grade adhesive, mechanical means, or the like. In particular, the proximal end **14** of the needle cannula **12** may be secured adhesively within the passageway **32** at the distal end **28** of the body **24** of the hub **22**. The proximal end **26** of the body **24** may include a tapered or reduced diameter portion **34** having a shoulder **36**, whose function will be discussed hereinafter. The proximal end **26** of the body **24** is generally adapted to cooperate with the tube **98** used to connect the medical device **10** to, for example, a blood collection receptacle. The passageway **32** at the distal end **28** of the body **24** preferably includes a reduced diameter portion or area **38** sized to accept and support the proximal end **14** of the needle cannula **12**.

The needle shield **40** includes a generally tubular body or housing **42** adapted to generally coaxially receive the hub **22,** as shown in FIG. 1. The needle shield **40** is preferably a unitary structure, which is desirably molded from a thermoplastic material. The body **42** of the needle shield **40** includes a proximal end **44** and a distal end **46**. The body **42** defines a central bore or passageway **48** extending between the proximal end **44** and distal end **46**. The passageway **48** is sized to coaxially receive the body **24** of the hub **22.** Preferably, an inner wall or surface **50** forming the passageway **48** substantially slidably cooperates with the outer surface **30** of the body **24** of the hub **22**, with only a small gap therebetween.

The needle shield **40** includes a butterfly-type wing assembly **52** including a pair of wings **54** extending laterally from the body **42** of the needle shield **40.** The pair of wings **54** extends from opposing sides of the body **42** for stabilizing the medical device **10** in contact with the body of a patient during a blood collection or other fluid collection procedure. In particular, the wings **54** assist in positioning and placing the medical device **10** and associated blood collection set during a blood collection procedure, for example. The wings **54** are adapted to lie flat against the surface of a patient's skin. The wings **54** may be constructed of a flexible material such that at least one, and preferably both, of the wings **54** may be bent toward each other and brought together between the fingers of the user to assist in positioning and placing the medical device **10** during venipuncture. The proximal end **44** of the body **42** of the needle shield **40** includes an outwardly bulged or tapered portion **56**, which defines one or more recesses or detents **58** for coacting with portions of the hub **22**, as discussed further hereinafter.

The needle shield **40** is generally movable between a first or retracted position coaxially surrounding the hub **22** and a second, extended or needle-shielding position, wherein the needle cannula **12** is enclosed or disposed within the needle shield **40** fully covering the needle cannula **12** and, in particular, the puncture tip **20**. The first or retracted position of the needle shield **40** is shown in FIG. 1 and the second or extended position of the needle shield **40** is shown in FIG. 2. In the first position of the needle shield **40,** the distal end **28** of the hub **22** preferably does not extend (i.e., project) beyond the distal end **46** of needle shield **40**. However, the distal end **16** of the needle cannula **12** projects outward from the needle shield **40** for use in a fluid collection procedure. Moreover, the inner surface **50** of the needle shield **40** is in substantial cooperating (i.e., slidable) engagement with the outer surface **30** of the hub **22,** as indicated previously.

The needle shield **40** is secured releasably in the first position by a release element **60**. The release element **60** is preferably fixed to the hub **22**, for example, by a medical grade adhesive or by mechanical methods. The release element **60** has a body **62** with a first or proximal end **64** and second or distal end **66**. The body **62** defines an internal bore or passageway **68** adapted to receive the hub **22**. The body **62** of the release element **60** is generally comprised of an annular-shaped inner member **70** defining the passageway **68**, and one or more locking arms **72** located about the inner member **70**. An annular groove or pocket **74** is formed or defined by the inner member **70**, the locking arms **72**, and the outer surface **30** of the hub **22**. The function of the annular groove or pocket **74** will be described hereinafter. While illustrated as a separate structure from the hub **22**, the release element **60** may be integrally formed as part of the hub **22.** The release element **60** is desirably molded from a thermoplastic material, either separate from or integrally with the hub **22** as indicated. Thus, the release element **60** may be considered to be part of the hub **22** in accordance with the present invention and generally forms a "grippable" or "graspable" portion of the hub **22,** which extends proximally out from the needle shield **40**. The release element **60** thus forms a convenient handle means for manipulating the medical device **10.** An outer surface **75** of the body **62** of the release element **60** and, more particularly, the locking arms **72** may be textured providing a surface grip for ease in grasping and manipulating the medical device **10** during a fluid collection procedure. When formed as a separate structure, the release element **60** in this embodiment of the medical device **10** is preferably fixedly connected to the hub **22**, for example by a medical grade adhesive or by mechanical methods, so that the locking arms **72** are positioned at fixed locations around the hub **22.**

The body **62** of the release element **60** generally coaxially receives the proximal end **26** of the body **24** of the hub **22,** as illustrated in FIGS. 1 and 2. In particular, the reduced diameter portion **34** at the proximal end **26** of the body **24** of the hub **22** is received in the annular-shaped inner member **70**, and preferably extends proximally outward from the proximal end **64** of the body **62** of the release element **60** to cooperate with the tube **98**. The inner member **70** defines a distal recess **76** opening to the internal passageway **68**. The distal recess **76** has a larger diameter than the diameter of the internal passageway **68**. The distal recess **76** is adapted to cooperate with the reduced diameter or tapered portion **34** defined by the body **24** of the hub **22**. The shoulder **36** of the reduced diameter portion **34** engages the distal recess **76** in the first position of the needle shield **40** and prevents axial movement of the release element **60** along the body **24** of the hub **22** in the distal direction, as is most apparent in FIG. 2.

The locking arms **72** extend distally along the inner member **70** and terminate in integrally formed barbs or hooks **78** adapted to mate with the detents **58** provided at the proximal end **44** of the needle shield **40**. In particular, as indicated previously, the detents **58** are formed in the outwardly bulged portion **56** at the proximal end **44** of the body **42** of the needle shield **40**. The detents **58** and locking arms **72** form a locking element of the medical device **10**. The engagement of the hooks **78** with the detents **58** secures the needle shield **40** in the first or retracted position. As indicated previously, the release element **60** is preferably fixedly connected to or integrally formed with the hub **22**. Thus, the locking arms **72** and, more particularly, the hooks **78** are positioned at fixed locations around the hub **22** opposite the detents **58** to properly engage the detents **58** and secure the needle shield **40** in the first or retracted position. It is preferred that the medical device **10** be preset by the manufacturer with the needle shield **40** in the first, shield-retracted position with a cover enclosing the puncture tip **20.** The user of the medical device **10** then only needs to make the appropriate connection to, for example, a blood collection receptacle, and remove the needle cover to make the medical device **10** ready for use.

As stated, the body **62** of the release element **60** is molded of a thermoplastic material, thereby making the locking arms **72** inherently flexible. The flexible locking arms **72** permit the locking arms **72** to be compress inward toward the hub **22**, which releases the hooks **78** of engagement with the detents **58** and enables the needle shield **40** to be moved to the second, needle-shielding position. The present invention envisions that once the locking arms **72** are released of engagement with the needle shield **40**, the needle shield **40** may be moved by the user of the medical device **10** to the second position or moved automatically to the second, needle-shielding position by means discussed hereinafter. It will be apparent that the location for the locking arms **72** and corresponding detents **58** may be reversed in accordance with the present invention. Accordingly, the locking arms **72** may be provided at the proximal end **26** of the body **24** of the hub **22** and extend proximally toward the release element **60**. The detents **58**, previously located on the hub **22**, may be provided at the distal end **66** of the body **62** of the release element **60**, such as defined in an annular outer member located coaxially out from and connected to the inner member **70**.

The medical device **10** preferably further includes a biasing member **80** adapted to act between the release element **60** formed with or connected to the hub **22** and the needle shield **40** to automatically (i.e., passively) move the needle shield **40** to the second, needle-shielding position. The biasing member **80** is preferably a compression spring or the like. The biasing member **80** is disposed in the pocket **74** defined by the inner member **60**, the locking arms **72** and outer surface **30** of the hub **22,** as shown in FIG. 1. The biasing member **80** is maintained in a compressed state by engagement of the hooks **78** of the locking arms **72** with the detents **58** in the needle shield **40**. The biasing member **80** is retained in a compressed state in the pocket **74** by contact or engagement with the proximal end **44** of the body **42** of the needle shield **40**. The distal end of the biasing member **80** may be secured to the proximal end **44** of the body **42** of the needle shield **40** by a suitable medical grade adhesive known in the art or by a fixed mechanical connection. Likewise, the proximal end of the biasing member **80** may be adhesively or mechanically secured in the pocket **74**. Once the biasing member **80** is released by disengaging the hooks **78** on the locking arms **72** from the corresponding detents **58**, the biasing member **80** exerts an axial expansion force against the needle shield **40**, and in particular, against the proximal end **44** of the needle shield **40**, relative to the hub **22** and release element **60**. The needle shield **40** is urged by the biasing member **80** to the second, needle-shielding position, wherein the needle shield **40** covers the needle cannula **12** (i.e., FIG. 2). The biasing member **80** also substantially prevents the needle shield **40** from moving proximally back toward the release element **60** and proximal end **44** of the body **42** of the needle shield **40** by providing a counter-acting biasing force against such a movement.

The medical device **10** further includes a locking element **82** provided at the distal end **28** of the body **24** of the hub **22.** In particular, the locking element **82** is preferably integrally formed as part of the distal end **28** of the body **24**. The locking element **82** preferably comprises two outward-biased locking arms **84** each having a barb or hook **85** adapted to cooperate with the detents **58** defined in the body **42** of the needle shield **40**, at the proximal end **44** of the needle shield **40**. The outward-biased locking arms **84** are restrained in the passageway **48** of the needle shield **40** when the needle shield **40** surrounds the hub **22** by contact with the inner surface **50** of the needle shield **40**. Once the biasing element **80** is released by the release element **60** and the needle shield **40** has begun moving to the second, needle-shielding position, the locking arms **84** slide along in contact with the inner surface **50** of the needle shield **40** until the hooks **85** reach the detents **58** formed in the needle shield **40**. The outward-biased locking elements **82** and, more particularly, the hooks **85** then snap into engagement with the detents **58** and prevent the needle shield **40** from moving proximally along the hub **22** toward the release element **60**, thereby preventing re-exposure of the needle cannula **12.**

Referring to FIGS. 1, 2, 3A, and 3B, the outer surface **30** of the hub **22** and the inner surface **50** of the needle shield **40** preferably interact such that any axial rotation of the hub **22** with respect to the needle shield **40** is inhibited during the sliding, expanding movement of the needle shield **40** from the first position to the second position. This ensures that the locking barbs or hooks **85** of the locking arms **84** mate properly with the detents **58** when the needle shield **40** is moved from the first to the second positions.

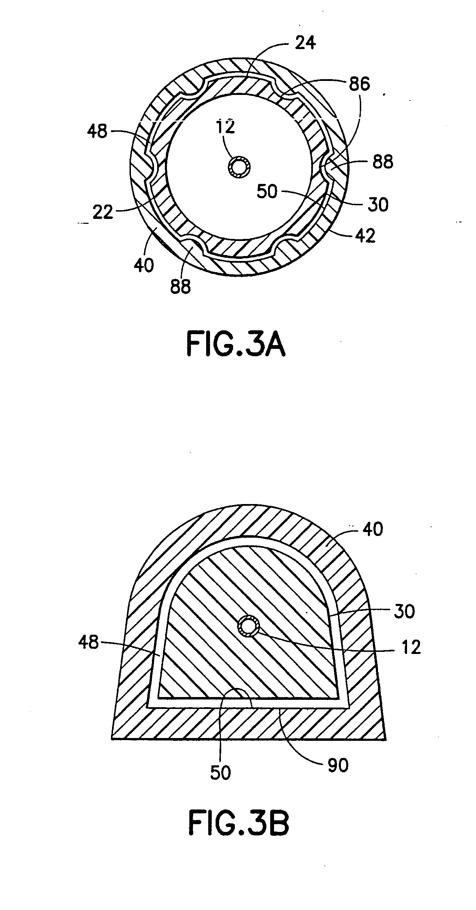

In one embodiment, the hub **22** and needle shield **40** may each have a substantially circular cross section as shown in FIG. 3A. To prevent rotation of the needle shield **40** relative to the hub **22**, the hub **22** defines at least one and, preferably, a plurality of spline grooves or detents **86** in the outer surface **30** of the body **24** of the hub **22**. The spline grooves or detents **86** mate with one or more splines **88** formed on the inner surface **50** of the body **42** of the shield member **40**. The interaction of the splines **88** and spline detents **86** prevents rotation of the needle shield **40** relative to the hub **22**, when the needle shield **40** is extended from the first to the second, needle-shielding position. The mating splines **88** and spline detents **86** may be replaced by any suitably equivalent surface feature on the outer surface **30** of the hub **22** and the inner surface **50** of the needle shield **40**, which prevents relative rotation between these structures when the needle shield **40** is extended to the second, needle-shielding position. Additionally, the locations of the splines **88** and spline detents **86** (i.e., surface features) may be reversed in accordance with the present invention.

In an alternative embodiment shown in FIG. 3B, the hub **22** and needle shield **40** may each have a non-circular cross section, for example, with one planar side **90**. As will be appreciated by those skilled in the art the non-circular cross sections of the hub **22** and needle shield **40** will prevent relative rotation between these elements. Any polygonal shaped structure would be suitable for the shape of the hub **22** and needle shield **40** in accordance with the embodiment of the medical device **10** shown in FIG. 3B.

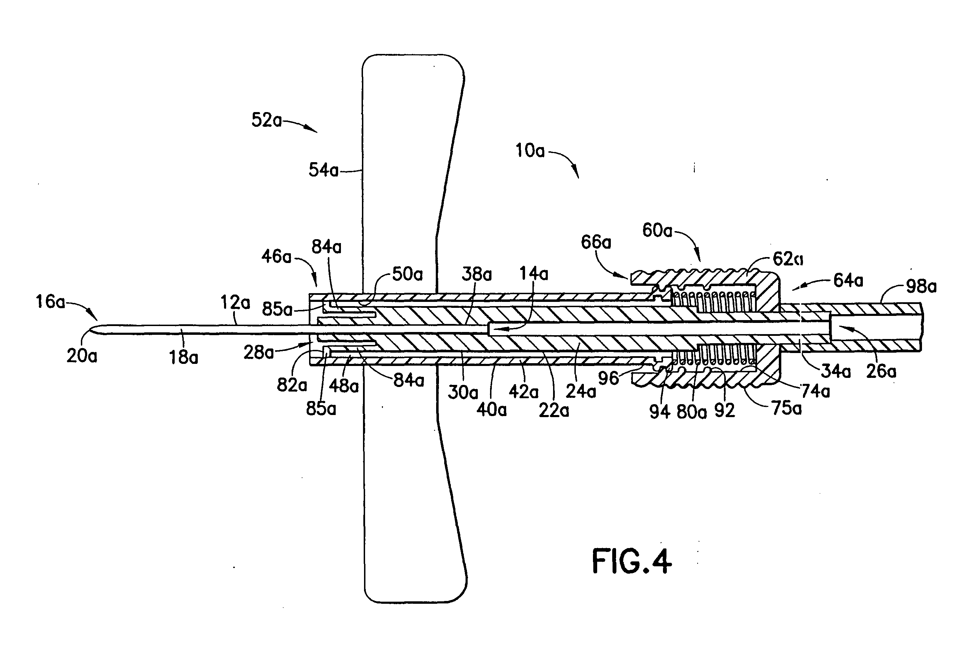

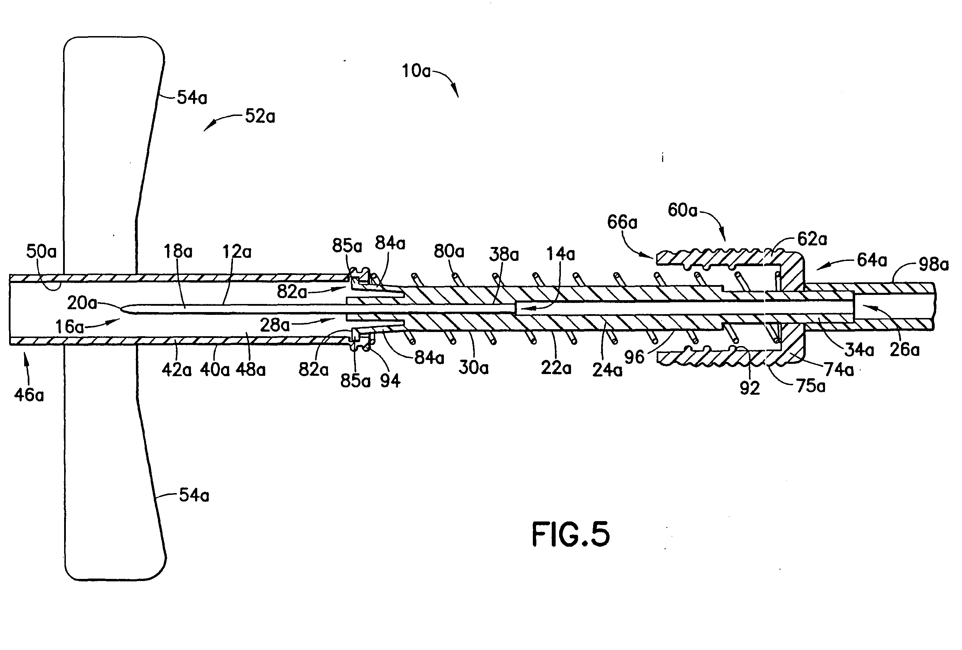

Another embodiment of the medical device **10a** is shown in FIGS. 4 and 5. The medical device **10a** of FIGS. 4 and 5 is substantially similar to the embodiment of the medical device **10** shown in FIGS. 1 and 2. However, the locking engagement between the release element **60a** and needle shield **40a** is slightly modified. The release element **60a** in this embodiment is provided as a rotatable locking collar that is received on the proximal end **26a** of the body **24a** of the hub **22a**. In particular, the rotatable release element **60a** is received about the reduced diameter portion **34a** at the proximal end **26a** of the body **24a** of the hub **22a**. The release element (i.e., rotatable locking collar) **60a** includes internal threads **92** adapted to cooperate with external threads **94** formed on an outer surface **96** of the body **42a** of the needle shield **40a**. The external threads **94** are provided at the proximal end **44a** of the body **42a** of the needle shield **40a.** The interaction between the internal and external threads **92, 94**, as shown in FIG. 4, prevents the needle shield **40a** from extending to the second, needle-shielding position. The needle shield **40a** is released to extend to the second, needle-shielding position by rotating the release element **60a** to unthread the release element **60a** from the proximal end **44a** of the needle shield **40a**. Once the release element **60a** is completely unthreaded from the needle shield **40a**, the biasing member **80a** automatically moves the needle shield **40a** to the second, needle-shielding position. The biasing member **80a** may be omitted and the user of the medical device **10a** may provided the necessary axial force to move the needle shield **40a** to the second, needle-shielding position. As will be apparent, the threaded locking arrangement between the release element **60a** and the needle shield **40a** may have a standard clockwise-to-thread, counter-clockwise-to-unthread configuration, or the opposite configuration. Rotation of the release element **60a** to unthread the release element **60a** from the needle shield **40a** will cause the needle shield **40** to begin to move distally toward the needle cannula **12** until engagement is released completely whereupon the biasing member **80a** moves the needle shield **40a** to the second, needle-shielding position automatically. The needle shield **40a** is locked in the second, needle-shielding position in the same manner as discussed previously in connection with the medical device **10** of FIGS. I and 2.

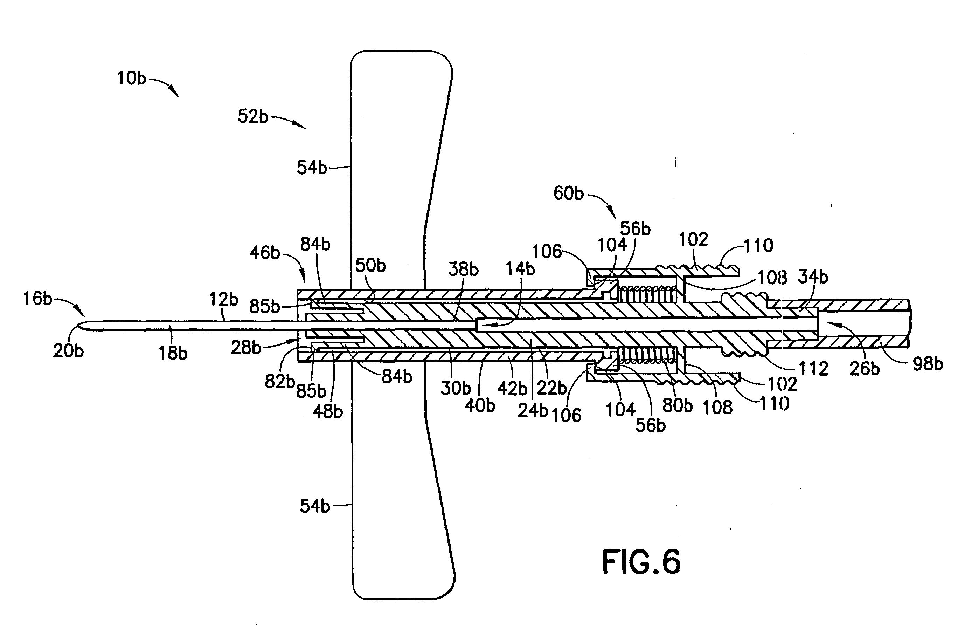

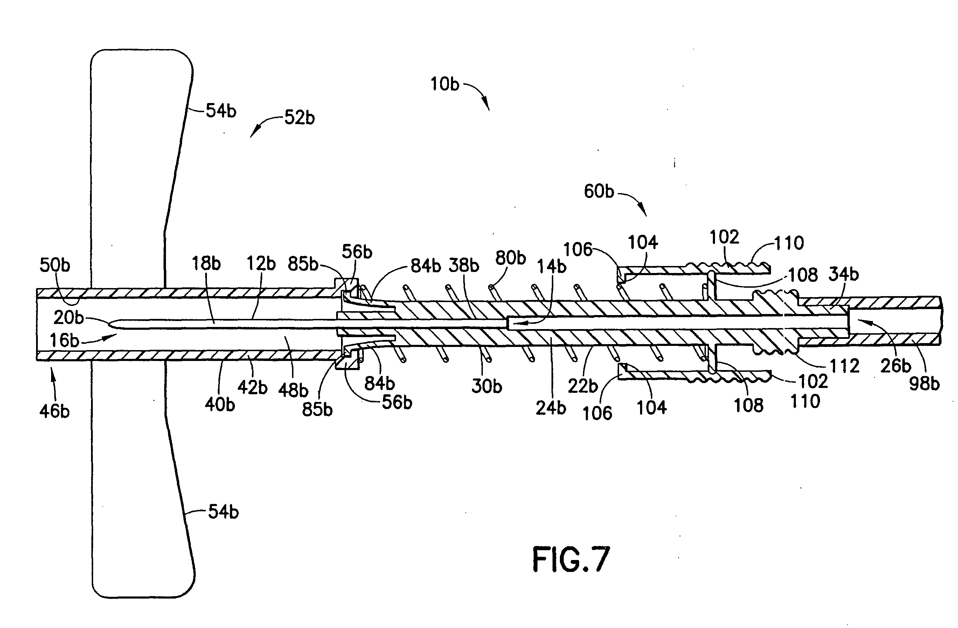

A further embodiment of the medical device **10b** of the present invention is shown in FIGS. 6 and 7. The medical device **10b** of FIGS. 6 and 7 is substantially similar to the embodiments of the medical device **10** shown in FIGS. 1, 2 and 4, 5. However, the locking engagement between the release element **60b** and needle shield **40b** is again slightly modified. The locking arms **72b** of the release element **60b** are now provided as pivot arms **102** adapted to mate with corresponding engagement surfaces **104** of the body **42b** of the needle shield **40**. The pivot arms **102** are pivotally connected to the body **24b** of the hub **22b**, at the proximal end **26b** of the body **24b**. The pivot arms **102** include hook portions or members **106** adapted to releasably mate with the corresponding engagement surfaces **104.** The engagement surfaces **104** are formed by the bulged portion **56b** of the body **42b** of the needle shield **40b**, as shown in FIG. 6. The bulged portion **56b** preferably now has a rectangular shape to form the engagement surfaces **104**. The interaction between the hook portions **106** of the pivot arms **102** and the engagement surfaces **104** at the proximal end **44b** of the needle shield **40b** prevents the needle shield **40b** from extending to the second, needle-shielding position. The needle shield **40b** is released to extend to the second, needle-shielding position by rotating or pivoting the pivot arms **102** about their respective pivot axes **108**, which may be, for example, hinges or fulcroms, which releases the hook portions **106** of engagement with the engagement surfaces **104.** The pivot arms **102** preferably include textured actuating members or tabs **110** for pivoting the pivot arms **102** and releasing their engagement with the engagement surfaces **104**. The texturing is provided to minimize the possibility that the user's fingers will slip from the pivot arms **102** when attempting to release the needle shield **40b**. Once the release element **60b** (i.e., pivot arms **102** and engagement surfaces **104**) is fully released, the biasing member **80b** automatically moves the needle shield **40b** to the second, needle-shielding position. The body **24b** of the hub **22b** may define a stop member **112** for limiting the axial distance that the tube **98** may extend along the proximal end **26b** of the body **24b** of the hub **22b**. The needle shield **40b** is locked in the second, needle-shielding position in the same manner as discussed previously in connection with the medical devices **10, 10a** of FIGS. 1, 2 and 4, 5, respectively.

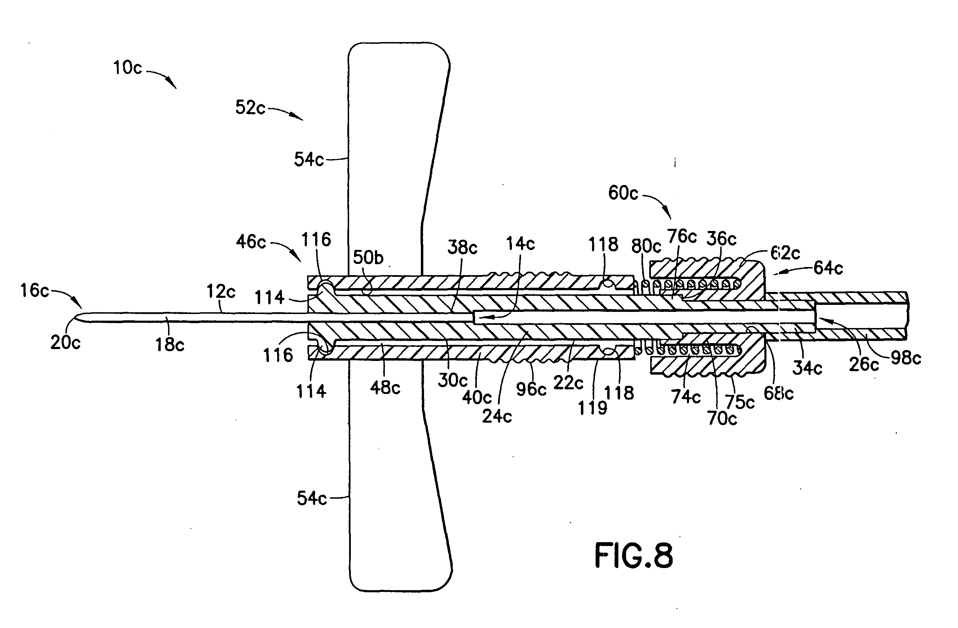

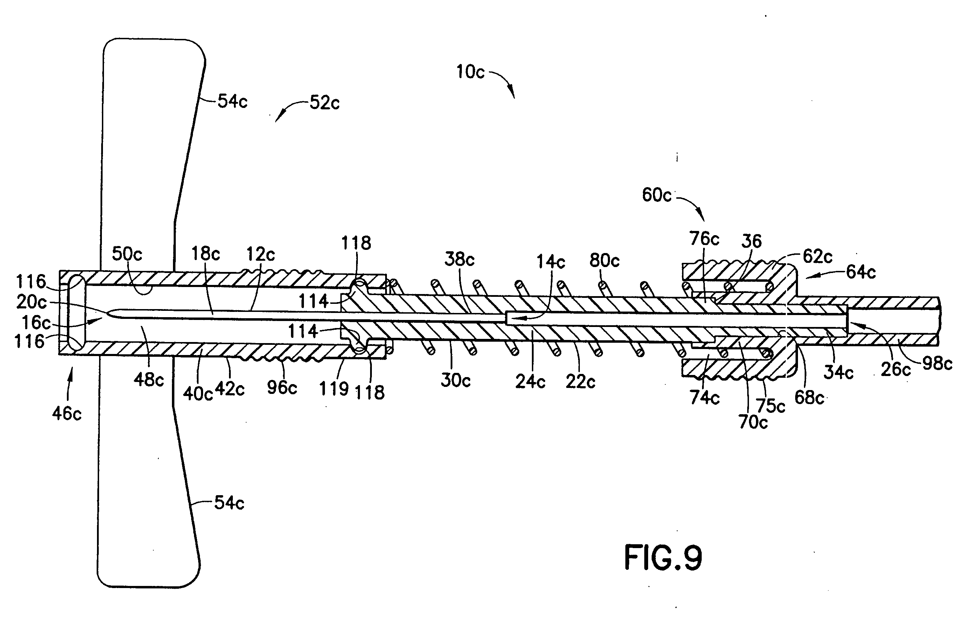

A final embodiment of the medical device **10c** is shown in FIGS. **8** and **9.** The medical device **10c** of FIGS. 8 and 9 is substantially similar to the embodiments of the medical device **10, 10a, 10b** shown in FIGS. 1, 2 and 4, 5 and 6, 7. However, the locking engagement between the release element **60c** and needle shield **40c** is replaced by a direct locking connection between the needle shield **40c** and hub **22c.** The release element **60c** may be provided as a rotatable collar in a similar manner to the medical device **10a** of FIGS. 4 and 5, or as a fixed and "grippable" element as discussed in connection with FIGS. 1 and 2. The release element **60c** houses the biasing member **80** in the manner discussed previously (i.e., in pocket **74c).**

The locking arms **84** of the locking element **82** provided at the distal end **28** of the hub **22** of the medical device **10** of FIGS. 1 and 2 are replaced in this embodiment with one or more raised members **114**, for example one or more splines or bumps. Alternatively, the raised members **114** may comprise a single raised member extending circumferentially around the distal end **28c** of the hub **22c**. The raised members **114** are preferably integrally formed as part of the body **24c** of the hub **22c,** or provided as an external structure added to the distal end **28c** of the body **24c.** The raised members **114** are adapted to cooperate with one or more corresponding detents or recesses **116** defined in the inner surface **50c** of the needle shield **40c** substantially at the distal end **46** of the needle shield **40**, and one or more corresponding detents or recesses **118** defined in the inner surface **50c** of the needle shield **40c** substantially at the proximal end **44** of the needle shield **40**. Alternatively, the detents or recesses **116, 118** may extend circumferentially around the inner surface **50c** of the needle shield **40c** (i.e., as a groove) when the raised members **114** are formed in a corresponding manner.

The interaction between the raised members **114**, or surface features or textures, and the corresponding detents **116, 118**, or surface features or textures, maintains the needle shield **40** in the first, retracted position or second, extended position. In particular, the raised members **114** will be received in the distal detents **116** when the needle shield **40c** is in the first or retracted position, and will be received in the proximal detents **118** when the needle shield **40c** is in the second or extended position. The frictional engagement of the raised member **114** and the distal detents **116** (i.e., corresponding or interacting surface features or textures) is preferably sufficient to prevent the biasing member **80c** from moving the needle shield **40c** to the second, needle-shielding position. In particular, the mutual friction between the outer surface **30c** of the hub **22c** (i.e., raised members **114**) and inner surface **50** of the needle shield **40c** (i.e., distal detents **116**) is greater than the biasing force of the biasing member **80**, which maintains the needle shield **40c** in the first position. For example, the mutual friction between the outer surface **30c** of the hub **22c** and inner surface **50c** of the needle shield **40c** may be about 120-1000% of the biasing force of the biasing member **80c.**

When the needle shield **40c** is to be extended to the second, needle-shielding position, the user of the medical device **10c** must urge or move the needle shield **40c** in a distal direction, which dislodges the raised members **114** from the distal detents **116.** The dislocation of the raised members **114** from the distal detents **116** reduces the frictional engagement between the hub **22c** and needle shield **40c**, which allows the biasing force exerted by the biasing member **80c** to move the needle shield **40c** automatically (i.e., passively) to the second, needle-shielding position. The biasing member **80c** interacts with the proximal end **26c** of the hub **22c** in the manner discussed previously to move the needle shield **40** to the needle-shielding position.

Once the biasing force of the biasing member **80c** is released, the needle shield **40c** moves distally to cover the needle cannula **12c**. The raised members **114** seat in the proximal detents **118** defined in the inner surface **50c** of needle shield **40c** at the proximal end **44c** of the needle shield **40c.** The frictional interaction between the outer surface **30c** of the hub **22c** (i.e., raised members **114**) and the inner surface **50c** of the needle shield **40c** (i.e., proximal detents **118)** and the biasing force of the biasing member **80c** prevents the needle shield **40c** from moving proximally and uncovering the needle cannula **12c**. Thus, an interference engagement between the hub **22c** and needle shield **40c** is used to prevent relative movement between these elements until the user of the medical device **10c** moves the needle shield **40c** distally to disengage the raised members **114** from the distal detents **116**, and further prevents the needle cannula **12c** from re-emerging from the needle shield **40c** once the needle shield **40c** is moved to the second position. The outer surface **96c** of the body **42c** of the needle shield **40c** may comprise a textured surface **119** forming a surface grip for grasping by the user of the medical device **10c.** In particular, in operation, the user of the medical device **10c** described hereinabove will apply opposing axial forces between the textured surface **119** of the needle shield **40c** and the textured surface **75c** of the release element **60c** (or hub **22c)** to overcome the mutual friction between the outer surface **30c** of the hub **22c** and the inner surface **50c** of the needle shield **40c** and release the biasing member **80c**, which then moves the needle shield **40c** to the second position automatically. The raised members **114** and corresponding distal and proximal detents **116, 118** may be replaced by any suitable mating or corresponding structures, surface features, or surface textures that exhibit sufficient mutual friction to overcome the biasing force of the biasing member **80c.**

As indicated previously, the medical device **10** of the present invention is preferably packaged with the needle shield **40** in the first position, as shown, for example, in FIGS. 1, 4, 6 and 8. Prior to use, the medical device **10** is removed from its package (not shown) for attachment with the flexible tube **98**, which is attached to an appropriate fluid collection receptacle. To prepare the medical device **10** for use with, for example, a blood collection set and other like devices, the user grasps the medical device **10** and removes the protective cover (not shown) to expose the puncture tip **20** of needle cannula **12**. The user then inserts the puncture tip **20** into a targeted blood vessel of a patient. During such positioning, at least one of the wings **54** may be bent inwardly toward the other by the user's fingers to facilitate positioning and placing the medical device **10.** Upon completion of the procedure, such as when all desired samples have been drawn, the needle cannula **12** is withdrawn from the patient. After removal of the needle cannula **12** from the patient, activation of the safety feature of the medical device **10** allows the compressed biasing member **80** to expand thereby urging the needle shield **40** to lock in the second, needle-shielding position in the manner discussed previously in connection with the various embodiments of the medical device **10.**

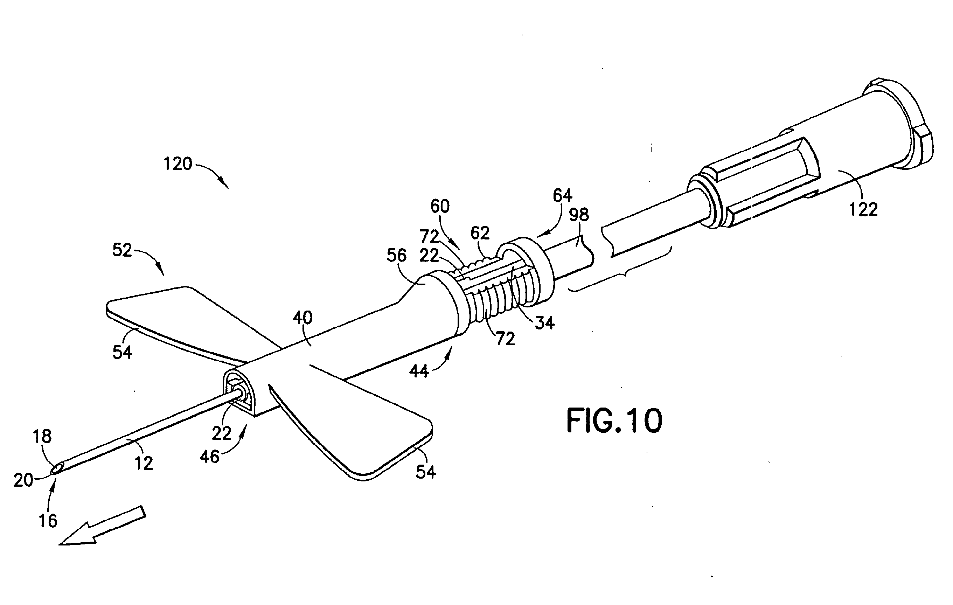

Referring to FIG. 10, the medical device of the present invention may be incorporated as part of a blood collection assembly **120,** as indicated previously. The tube **98** is connected to the proximal end **26** of the hub **22** is further connected to a blood collection receptacle **122**. The blood collection receptacle **122** is adapted to receive a fluid collection tub (not shown) such as a blood collection tube.

While the medical device of the present invention was described in terms of several preferred embodiments for use in connection with bodily fluid collection systems and like devices, the present invention may take many different forms. The preferred embodiments shown in the drawings and described hereinabove in detail are to be considered as exemplary of the principles of the invention and are not intended to limit the invention to the embodiments illustrated. Various other embodiments will be apparent to and readily made by those skilled in the art without departing from the scope and spirit of the invention. The scope of the present invention will be measured by the appended claims and their equivalents.

## Claims

1. A medical device, comprising:
a needle cannula;
a hub comprising a proximal end, a distal end and a passageway extending therethrough in fluid communication with the needle cannula, the distal end of the hub supporting the needle cannula;
a release element fixed to the hub;
a generally hollow needle shield comprising a proximal end, a distal end and a pair of wings extending laterally from opposing sides thereof, the proximal end of the needle shield removably secured to the release element with at least a grippable portion of the hub extending out from the proximal end of the needle shield, the needle shield axially movable with respect to the needle cannula from a first position in which the needle cannula is exposed to a second position in which the needle cannula is shielded within the needle shield; and
a biasing member extending between the distal end of the hub and the proximal end of the needle shield,
wherein activation of the release element releases the proximal end of the needle shield from securement, thereby causing the biasing element to axially urge the needle shield from the first position toward the second position.

2. A medical device as in claim 1, wherein the biasing member is a spring.

3. A medical device as in claim 1, wherein the outer surface of the hub and the inner surface of the needle shield interact such that axial rotation of the hub with respect to the needle shield is inhibited during movement of the needle shield from the first position toward the second position.

4. A medical device as in claim 3, wherein the hub and the needle shield have non-circular cross-sectional shapes, thereby inhibiting axial rotation of the hub with respect to the needle shield during movement of the needle shield from the first position toward the second position.

5. A medical device as in claim 3, wherein the inner surface of the needle shield includes splines for inhibiting axial rotation of the hub with respect to the needle shield during movement of the needle shield from the first position toward the second position.

6. A medical device as in claim 1, further comprising a flexible tube extending from the proximal end of the hub.

7. A medical device as in claim 1, further comprising a locking element for securing the needle shield in the second position.

8. A medical device as in claim 7, wherein the locking element comprises a pair of flexible locking arms at the distal end of the hub, each of the locking arms including a hook for releasable engagement with a corresponding detent in the proximal end of the needle shield.

9. A medical device as in claim 1, wherein the release element comprises at least one flexible locking arm extending distally from the hub and including a hook for releasable engagement with a corresponding detent in the proximal end of the needle shield.

10. A medical device as in claim 9, wherein the release element comprises a pair of flexible locking arms extending distally from the hub, each of the locking arms including a hook for releasable engagement with a corresponding detent in the proximal end of the needle shield.

11. A medical device as in claim 9, wherein the at least one flexible locking arm extends distally from the hub to form a pocket between the flexible locking arm and an outer surface of the hub adjacent the distal end of the hub, and wherein the pocket accommodates the biasing member.

12. A medical device as in claim 9, further comprising a locking element for securing the needle shield in the second position, the locking element comprising at least one flexible locking arm at the distal end of the hub and including a hook for releasable engagement with the detent in the proximal end of the needle shield when the needle shield is in the second position.

13. A medical device as in claim 1, wherein the release element comprises a rotational locking engagement between the distal end of the hub and the proximal end of the needle shield.

14. A medical device as in claim 13, further comprising a rotatable collar extending from the distal end of the hub and independently rotatable with respect to the hub, the rotatable collar in releasable engagement with the proximal end of the needle shield when the needle shield is in the first position.

15. A medical device as in claim 13, wherein the rotational locking engagement comprises a threaded engagement.

16. A medical device as in claim 1, wherein the release element comprises at least one pivot arm extending laterally along the hub, the pivot arm in releasable engagement with the proximal end of the needle shield.

17. A medical device as in claim 16, wherein the release element comprises a pair of pivot arms extending laterally along opposing outer sides of the hub, the pair of pivot arms in releasable engagement with corresponding surfaces on the proximal end of the needle shield.

18. A medical device, comprising:
a needle cannula;
a hub comprising a proximal end, a distal end and a passageway extending therethrough in fluid communication with the needle cannula, the distal end of the hub supporting the needle cannula;
a generally hollow needle shield comprising a proximal end, a distal end and a pair of wings extending laterally from opposing sides thereof, the needle shield axially movable with respect to the needle cannula from a first position in which the needle cannula is exposed to a second position in which the needle cannula is shielded within the needle shield; and
a release element fixed to the hub and removably retaining the proximal end of the needle shield with at least a portion of the hub extending from the proximal end of the needle shield, the release element comprising a rotational locking engagement the hub and the proximal end of the needle shield,
wherein activation of the release element releases the proximal end of the needle shield for movement of the needle shield from the first position toward the second position.

19. A medical device as in claim 18, wherein the rotational locking engagement comprises a threaded engagement.

20. A medical device as in claim 18, wherein the release element comprises a rotatable collar extending from the end of the hub and independently rotatable with respect to the hub, the rotatable collar in releasable engagement with the proximal end of the needle shield when the needle shield is in the first position.

21. A medical device as in claim 18, further comprising a biasing member extending between the distal end of the hub and the proximal end of the needle shield, for automatically urging the needle shield from the first position toward the second position upon activation of the release element.

22. A medical device, comprising:
a needle cannula;
a hub comprising a proximal end, a distal end and a passageway extending therethrough in fluid communication with the needle cannula, the distal end of the hub supporting the needle cannula;
a generally hollow needle shield comprising a proximal end, a distal end and a pair of wings extending laterally from opposing sides thereof, the needle shield encompassing the hub with at least a portion of the hub extending from the proximal end of the needle shield, the needle shield axially movable with respect to the needle cannula from a first position in which the needle cannula is exposed to a second position in which the needle cannula is shielded within the needle shield; and
a biasing member extending between the hub and the needle shield and exerting a biasing force to urge the needle shield distally toward the second position,
wherein when the needle shield is in the first position, the shield and the hub exhibit a mutual friction greater than the biasing force exerted by the biasing member when the needle shield is in the first position.

23. A medical device as in claim 22, wherein the mutual friction between the shield and the hub is about 120% to about 1000% of the biasing force exerted by the biasing element.

24. A medical device as in claim 22, wherein the mutual friction is provided through surface texture on at least one of an outside surface of the hub or an inside surface of the needle shield.

25. A medical device as in claim 22, wherein the portion of the hub which extends from the proximal end of the needle shield includes a surface grip and the needle shield includes a surface grip, such that opposing forces applied against the surface grip of the hub and the surface grip of the needle shield releases the mutual friction between the hub and the shield, and the biasing element causes axial movement of the needle shield toward the second position.

26. A medical device as in claim 22, further comprising a locking element for securing the needle shield in the second position.

27. A medical device as in claim 26, wherein an inner surface of the needle shield includes a first surface feature at the proximal end thereof and a second surface feature at the distal end thereof and the external surface of the hub includes a surface feature for interference engagement with the first surface feature of the needle shield when the needle shield is in the first position for retaining the needle shield in the first position, and for interference engagement with the second surface feature of the needle shield when the needle shield is the second position for securing the needle shield in the second position.

28. A medical device as in claim 26, wherein the first and second surface features of the needle shield comprise first and second detents, respectively, and the surface feature of the hub comprises a bump for respective interference engagement with the first and second detents of the needle shield.
